(19) Europäisches Patentamt
European Patent Office
Office européen des brevets

(11) Numéro de publication: **0 299 893**
**A2**

(12) **DEMANDE DE BREVET EUROPEEN**

(21) Numéro de dépôt: **88420245.8**

(22) Date de dépôt: **13.07.88**

(51) Int. Cl.⁴: **C 07 C 37/60**
C 07 C 41/26, B 01 J 29/28

(30) Priorité: **17.07.87 FR 8710420**

(43) Date de publication de la demande:
**18.01.89 Bulletin 89/03**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Costantini, Michel**
**10, rue du Docteur Bonhomme**
**F-69003 Lyon (FR)**

**Popa, Jean-Michel**
**2, rue Roger Breton**
**F-93700 Drancy (FR)**

(74) Mandataire: **Vignally, Noel et al**
**Rhône-Poulenc Interservices Service Brevets Chimie**
**Centre de Recherches des Carrières B.P. 62**
**F-69192 Saint-Fons Cédex (FR)**

(54) **Procédé d'hydroxylation de phénols et d'éthers de phénols.**

(57) La présente invention concerne un procédé d'hydroxylation de phénols ou d'éthers de phénols par le peroxyde d'hydrogène.

Plus précisément elle consiste en un procédé d'hydroxylation de phénols ou d'éthers de phénols par réaction avec le peroxyde d'hydrogène, caractérisé en ce que la réaction est conduite en présence d'une quantité efficace d'argile pontée.

EP 0 299 893 A2

**Description**

**PROCEDE D'HYDROXYLATION DE PHENOLS ET D'ETHERS DE PHENOLS**

La présente invention concerne un procédé d'hydroxylation de phénols ou d'éthers de phénols par le peroxyde d'hydrogène.

L'hydroxylation du phénol ou de phénols substitués, par le peroxyde d'hydrogènes, pour préparer des diphénols est une réaction connue.

Le brevet français n° 69-45467 publié sous le n° 2 071 464 a décrit un procédé dans lequel la réaction est catalysée par un acide fort, tel que par exemple l'acide perchlorique ou l'acide sulfurique.

Le brevet allemand n° 2 410 742 a décrit un procédé semblable au précédent, dans lequel le peroxyde d'hydrogène est mis en oeuvre sous forme de solution organique pratiquement anhydre.

Ces deux procédés sont très intéressants et le premier procédé est mis en oeuvre industriellement.

Cependant depuis quelques années, on cherche à catalyser la réaction d'hydroxylation à l'aide de solides non dissous dans le milieu, afin de simplifier leur séparation du milieu réactionnel et leur éventuel recyclage et éviter les sous-produits salins, que se forment le plus souvent lors de l'élimination des catalyseurs acides dissous.

Ainsi, la demande de brevet français n° 81-17023 (publiée sous le n° 2 489 816) préconise l'emploi de silicalite de titane comme catalyseur hétérogène de l'hydroxylation de composés aromatiques par le peroxyde d'hydrogène.

La fine taille des particules de catalyseur utilisées rend leur séparation du milieu réactionnel très difficile et leur recyclage problématique, alors que dans un procédé industriel, il est indispensable de pouvoir recycler un catalyseur coûteux.

Pour remédier à ce problème de la séparation du catalyseur, il a été proposé, dans la demande de brevet européen publiée sous le n° 200 260, d'utiliser des agglomérats de ces fines particules de silicalite de titane.

Il s'avère cependant que l'on recherche encore une catalyse hétérogène de la réaction d'hydroxylation des phénols ou éthers de phénols par le peroxyde d'hydrogène, qui puisse être utilisée de manière industrielle dans des conditions économiquement acceptables. C'est précisément l'objet de la présente invention.

L'invention consiste donc en un procédé d'hydroxylation de phénols ou éthers de phénols de formule générale (I) :

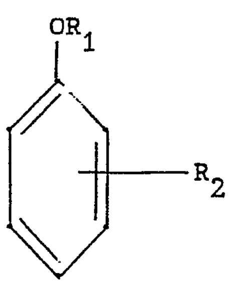

(I)

dans laquelle :

- $R_1$ représente un atome d'hydrogène, un groupement méthyle, un groupement éthyle ou un groupement phényle,
- $R_2$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace d'au moins une argile pontée.

Par argile pontée, on entend dans le présent texte des argiles entre les feuillets desquelles ont été introduits des ponts ou piliers qui maintiennent un espacement basal. L'espacement basal (ou distance interlamellaire) est la somme de l'épaisseur d'un feuillet de l'argile et de l'espacement interfoliaire.

Généralement les argiles pontées utilisées dans le procédé d'invention ont un espacement basal supérieur à 10 Angstroms (1 nm).

Les argiles pontées sont par exemple les zéolithes à structure bidimensionnelle qui sont préparées selon le procédé de pontage décrit dans la demande de brevet français n° 84-06482 (publiée sous le numéro 2 563 446). Ces zéolithes à structure bidimensionnelle sont obtenues par un procédé comportant les étapes suivantes :

a) traitement d'une argile naturelle ou synthétique, sous forme d'une suspension aqueuse, par une solution aqueuse contenant au moins un hydroxyde d'au moins un métal ;

b) l'élimination de l'excès de l'hydroxyde de métal n'ayant pas réagi sur l'argile, ladite élimination conduisant, après séchage de l'argile traitée, à l'obtention d'un précurseur de la zéolithe ;

c) le traitement thermique du précurseur de la zéolithe, ledit traitement conduisant à l'obtention de la zéolithe à structure bidimensionnelle.

Pour la préparation d'argile pontée utilisable dans le cadre de l'invention, on peut également ne mettre en oeuvre que les étapes a) et b) précédentes.

Les argiles qui peuvent être soumises à un traitement de pontage, sont notamment les smectites. Parmi ces smectites, on peut citer à titre d'exemples non limitatifs les beidellites, en particulier les beidellites synthétiques, et les montmorillonites.

La préparation des beidellites synthétiques qui seront ainsi pontées est elle-même décrite dans la demande de brevet précité et dans des articles auxquels se réfère ladite demande de brevet.

Le pontage des argiles peut être effectué à l'aide des hydroxydes des métaux suivants : l'aluminium, le nickel, le cobalt, le vanadium, le molybdène, le rhénium, le fer, le cuivre, le ruthénium, le chrome, le lanthane, le cérium, le titane, le bore, le gallium, le zirconium, le nobium, le tantale, le silicium, ainsi qu'avec des hydroxydes mixtes de ces métaux.

De préférence on utilisera comme catalyseur dans le présent procédé une beidellite ou une montmorillonite pontée à l'aide d'hydroxyde d'aluminium, par exemple selon le procédé de la demande de brevet FR 84-06482.

Les phénols et éthers de phénols qui sont utilisés de préférence dans le procédé de l'invention sont les composés de formule (I) dans laquelle $R_1$ représente un atome d'hydrogène, un groupment méthyle ou un groupement éthyle, et $R_2$ représente un atome d'hydrogène, un groupement méthyle, éthyle ou tertiobutyle, un groupement méthoxy ou éthoxy.

A titre d'exemple non limitatifs on peut citer le phénol, l'anisole, l'orthocrésol, le métacrésol, le paracrésol, le tertiobutyl-4 phénol, le méthoxy-2 phénol, le méthoxy-4 phénol.

Le procédé selon l'invention s'applique particulièrement bien au phénol pour la préparation d'hydroquinone et de pyrocatéchine.

Le peroxyde d'hydrogène peut être utilisé sous la forme d'une solution aqueuse ayant généralement une concentration en peroxyde d'hydrogène supérieure à 20 % en poids. Le peroxyde d'hydrogène peut également être utilisé sous la forme d'une solution dans un solvent organique. Comme solvants organiques utilisables pour la mise en oeuvre du peroxyde d'hydrogène, on peut utiliser des esters tels que notamment les esters d'alkyle ou de cycloalkyle d'acides carboxyliques aliphatiques saturés ; de préférence on emploiera les acétates et les propionates d'alkyle ayant au total de 4 à 8 atomes de carbone ou des mélanges de tels esters. On peut également utiliser des solutions de peroxyde d'hydrogène dans un éther tel que par exemple le dioxanne, le diisopropyléther ou le méthyl- tertiobutyléther.

Le rapport molaire phénols ou éthers de phénols de formule I/peroxyde d'hydrogène est généralement de 25/1 à 3/1 et préférentiellement de 20/1 à 4/1. La quantité d'argile pontée, et plus particulièrement de zéolithe à structure bidimensionelle définie précédemment, que l'on peut mettre en oeuvre dans le présent procédé peut varier dans de très larges limites.

Lorsque l'on réalise le procédé en discontinu, le catalyseur peut représenter en poids par rapport au poids du composé de formule I engagé de 0,1 % à 20 %. De préférence, ce rapport pondéral sera compris entre 0,5 % et 10 %. Cependant si l'on réalise le procédé en continu, par exemple en faisant réagir un mélange de composé de formule I et de solution de peroxyde d'hydrogène sur un lit fixe de catalyseur, ces rapports catalyseur/composé de formule I n'ont plus de sens et, à un instant donné, on pourra avoir un excès pondéral de catalyseur par rapport au composé de formule I.

Il est également possible d'effectuer le réaction d'hydroxylation du composé I dans un solvant dudit composé I, qui soit de préférence miscible ou partiellement miscible à l'eau.

A titre d'exemples non limitatifs de tels solvants, on peut citer l'eau ; les alcools comme le méthanol, le tertiobutanol, l'isopropanol ou l'éthanol ; les cétones comme l'acétone ou la méthylisobutylcétone ; les nitriles comme l'acétonitril ; les acides carboxyliques comme l'acide acétique ; les esters comme l'acétate de propyle ; les éthers comme le méthyl-tertiobutyléther ; des solvants aprotiques polaires comme le dioxyde de tétrahydrothiophène (sulfolane), le carbonate d'éthylène glycol, le carbonate de propylène glycol, la N-methylpyrrolidone.

On peut également rajouter un composé phosphoré, tel qu'un acide phosphorique, un acide phosphonique ou l'un de leurs dérivés. Généralement ces composés permettent d'améliorer les rendements en composé hydroxylé par rapport au peroxyde d'hydrogène transformé. Généralement on utilise de 0 à 10 % en mole de composé phosphoré par rapport au peroxyde d'hydrogène.

La température à laquelle est conduite la réaction est généralement comprise entre 45° et 160°C sous pression atmosphérique. On peut également opérer à plus haute température et sous pression supérieure à la pression atmosphérique.

Les exemples qui suivent illustrent l'invention.

EXEMPLE 1 : PREPARATION DU CATALYSEUR TRP-1 (selon le procédé de la demande de brevet FR 84-06482)

La beidellite est une argile TOT, silicoaluminate de type smectite dioctaédrique, dont une partie des atomes de silicium de la couche tétraédrique est substituée par des atomes d'aluminium.

Le beidellite pontée est obtenue par pontage, à l'aide d'une solution d'hydroxyde d'aluminium, d'une beidellite sodique.

La solution de polyoxycations d'aluminium est préparée par ajout d'ammoniaque à une solution de nitrate d'aluminium jusqu'à un rapport OH/A1 de 1,2 (pH = 4). Cette solution est ensuite chauffée pendant 1 heure

50°C.

On prépare une dispersion dans l'eau de la beidellite sodique à laquelle on ajoute la solution précédente. Le mélange des deux solutions est vigoureusement agité pendant 1 heure à température ambiante. La solution ainsi obtenue est dialysée pendant quatre jours.

Le matériau est ensuite filtré, puis séché à 100°C pendant deux heures. Le produit séché est ensuite calciné à 350°C.

Le matériau TRP-1 ainsi obtenu est caractérisé par une distance interlamellaire (ou espacement basal) après calcination à 350°C de 17,6 Angstroms (1,76 nm), par une surface spécifique de 420 m2/g et par un volume poreux de 0,43 cm3/g.

La beidellite pontée obtenue se présente sous la forme de petits cylindres d'environ 1 à 5 millimètres de longueur et d'environ 0,5 à 1 millimètre de diamètre.

EXEMPLE 2

Dans un réacteur en verre pyrex de 100 cm3, muni d'une agitation centrale, d'un réfrigérant relié à un gazomètre, d'une système de chauffage régulé et d'un système d'injection, on charge après avoir préalablement purgé l'appareil à l'aide d'azote :

- 27,2 g de phénol
- 0,528 g de beidellite pontée préparée dans l'exemple 1.

On chauffe à 80°c sous agitation, puis on injecte en 1 heure 6,32 g d'une solution de $H_2O_2$ à 40 % en poids par volume.

On laisse ensuite réagir pendant 1 heure supplémentaire.

Après filtration du catalyseur, on dose $H_2O_2$ non transformée par iodométrie et les diphénols par chromatographie liquide haute performance (CLHP).

On obtient les résultats suivants :

- taux de transformation (TT)de $H_2O_2$ : 31,5 %
- rendement en pyrocatéchine par rapport à $H_2O_2$ transformée (RT) : 27,5 %
- rendement en hydroquinone par rapport à $H_2O_2$ transformée (RT) : 12,5 %
- rendement total en diphénols : 40,0 %

EXEMPLE 3

Dans l'appareillage décrit pour l'exemple 2, en suivant le même mode opératoire et avec les mêmes charges de réactifs, on réalise un nouvel essai en laissant réagir pendant 5 h 30 min (au lieu d'une heure) après la fin de la coulée de $H_2O_2$.

On dose $H_2O_2$ non transformée par iodométrie et les diphénols formés par CLHP.

On obtient les résultats suivants :

- taux de transformations (TT)de $H_2O_2$ : 100,0 %
- rendement en pyrocatéchine par rapport à $H_2O_2$ transformée (RT) : 10,0 %
- rendement en hydroquinone par rapport à $H_2O_2$ transformée (RT) : 8,5 %
- rendement total en diphénols : 18,5 %

EXEMPLE 4

On répète l'exemple 2 en chargeant 2 g du catalyseur préparé dans l'exemple 1.

On obtient les résultats suivants :

- taux de transformation (TT)de $H_2O_2$ : 81,0 %
- rendement en pyrocatéchine par rapport à $H_2O_2$ transformée (RT) : 8,0 %
- rendement en hydroquinone par rapport à $H_2O_2$ transformée (RT) : 5,0 %
- rendement total en diphénols : 13,0 %

EXEMPLE 5

On répète l'exemple 2 avec les différences suivantes :

- 2 g du catalyseur de l'exemple 1
- 1,45 g de solution aqueuse de $H_2O_2$ à 40 % en poids par volume (p/v)
- agitation pendant 1 h 30 min après la fin de coulée de $H_2O_2$.

On obtient les résultats suivants :

- taux de transformation (TT)de $H_2O_2$ : 97,5 %
- rendement en pyrocatéchine par rapport à $H_2O_2$ transformée (RT) : 9,0 %
- rendement en hydroquinone par rapport à $H_2O_2$ transformée (RT) : 8,0 %
- rendement total en diphénols : 17,0 %

EXEMPLE 6

Dans un réacteur en verre pyrex de 30 cm3, muni d'une agitation magnétique, d'un réfrigérant relié à un gazomètre, d'un système de chauffage regulé et d'un système d'injection, on charge après avoir préalablement purgé l'appareil à l'aide d'azote :
- 9,4 g de phénol
- 0,25 g de beidellite pontée préparée dans l'exemple 1.

On chauffe à 80° C sous agitation, puis on injecte en 0,1 min 0,25 g d'une solution de $H_2O_2$ à 70 % en poids par volume (5 mmol).

On laisse ensuite réagir pendant 2 h 30 min supplémentaires.

Après filtration du catalyseur, on dose $H_2O_2$ non transformée par iodométrie et les diphénols par chromatographie liquide haute performance (CLHP).

On obtient les résultats suivants :
- taux de transformations (TT)de $H_2O_2$ : 71,2 %
- rendement en pyrocatéchine par rapport à $H_2O_2$ transformée (RT) : 12,9 %
- rendement en hydroquinone par rapport à $H_2O_2$ transformée (RT) : 5,9 %
- rendement total en diphénols : 18,8 %

EXEMPLE 7

On répète l'exemple 6 avec la différence suivante :
- 0,5 g du catalyseur de l'exemple 1.

On obtient les résultats suivants :
- taux de transformation (TT)de $H_2O_2$ : 61,6 %
- rendement en pyrocatéchine par rapport à $H_2O_2$ transformée (RT) : 16,4 %
- rendement en hydroquinone par rapport à $H_2O_2$ transformée (RT) : 8,9 %
- rendement total en diphénols : 25,3 %

EXEMPLES 8 A 15

Dans un réacteur en verre pyrex de 30 cm3, muni d'une agitation magnétique, d'un réfrigérant relié à un gazomètre, d'un système de chauffage régulé et d'un système d'injection, on charge après avoir préalablement purgé l'appareil à l'aide d'azote :
- 4,7 g de phénol
- 0,25 de beidellite pontée préparée dans l'exemple 1.
- 4,7 g d'un solvant organique (voir tableau ci-après).

On chauffe à 80° C sous agitation, puis on injecte en 0,1 min 0,125 g d'une solution de $H_2O_2$ à 70 % en poids par volume (2,5 mmol).

On laisse ensuite réagir pendant 2 h 30 min supplémentaires.

Après filtration du catalyseur, on dose $H_2O_2$ non transformée par iodométrie et les diphénols par chromatographie liquide haute performance (CLHP).

Le tableau I ci-après rassemble les résultats obtenus (pyrocatéchine = PC ; hydroquinone = HQ).

TABLEAU I

| EXEMPLES | SOLVANTS | TT % H2O2 | RT % HQ | RT % PC | Somme RT % |
|---|---|---|---|---|---|
| Ex. 8 | acide acétique | 96,1 | 6,5 | 1,9 | 8,4 |
| Ex. 9 | acétonitrile | 17,8 | 4,8 | 11,5 | 16,3 |
| Ex. 10 | méthyl-isobutylcétone | 22,3 | 62,3 | 33,4 | 95,7 |
| Ex. 11 | Acétate de propyle | 18,9 | 5,9 | 10,1 | 16,0 |
| Ex. 12 | tertiobutanol | 8,8 | 2,0 | 3,7 | 5,7 |
| Ex. 13 | Méthanol | 6,2 | 12,1 | 4,6 | 16,7 |
| Ex. 14 | sulfolane | 26,4 | 11,3 | 13,0 | 24,3 |
| Ex. 15 | eau | 6,6 | 0 | 7,1 | 7,1 |

EXEMPLES 16 à 18

Dans un réacteur en verre pyrex de 30 cm3, muni d'une agitation magnétique, d'un réfrigérant relié à un gazomètre, d'un système de chauffage régulé et d'un système d'injection, on charge après avoir préalablement purgé l'appareil à l'aide d'azote :

- 9,4 g de phénol
- 0,25 g de beidellite pontée TRP-1 préparée dans l'exemple 1 (ayant subi éventuellement un traitement complémentaire).

On chauffe à 80° C ou à 130° C sous agitation, puis on injecte en 0,1 min 0,25 g d'une solution de $H_2O_2$ à 70 % en poids par volume (5 mmol).

On laisse ensuite réagir pendant 2 h 30 min supplémentaires.

Après filtration du catalyseur, on dose $H_2O_2$ non transformée par iodométrie et les diphénols par chromatographie liquide haute performance (CLHP).

Le tableau II ci-après rassemble les résultats obtenus (pyrocatéchine = PC ; hydroquinone = HQ).

TABLEAU II

| Exemples | Traitement complémentaire du catalyseur | T°C | TT % H2O2 | RT % HQ | RT % PC | Somme RT % des diphénols |
|---|---|---|---|---|---|---|
| Ex. 16 | calciné 5 h à 550°C | 80 | 50,0 | 11,0 | 23,0 | 34,0 |
| Ex. 17 * | néant | 80 | 34,5 | 12,5 | 16,5 | 29,0 |
| Ex. 18 | néant | 130 | 79,5 | 14,0 | 24,0 | 38,0 |

* essai effectué en présence de 4 % en mole de diphosphono-1,1 éthanol (DPE) par rapport à $H_2O_2$

EXEMPLE 19 : Exemples de pontage d'une montmorillonite.

On utilise pour ces essais de pontage une montmorillonite du Wyoming, aussi appelée bentonite Volclay.

Préparation de la fraction à 2 μm de l'argile

On prépare une suspension aqueuse à 20 g/litre de cette argile, qu'on agite violemment et qu'on laisse décanter pendant 24 heures.

On fait ensuite un prélèvement de la fraction à 2 μm de l'argile (solution a).

Préparation de l'argile sous forme sodique

On ajoute à la solution a du chlorure de sodium en excès, par rapport à la capacité d'échange de l'argile, soit environ 1000 milliéquivalents de sodium pour 100 g d'argile. La solution est agitée pendant 1 heure à température ambiante. L'argile est alors filtrée de la solution. On recommence l'opération une deuxième fois. L'argile est ensuite lavée à l'eau distillée jusqu'à disparition des ions chlorures dans les eaux de lavage. L'argile est alors remise en suspension dans l'eau à 20 g/litre (solution b).

Préparation de la solution d'hydroxyde d'aluminium

On prépare une solution 0,4 M de nitrate d'aluminium, à laquelle on ajoute, sous agitation et à température ambiante, une solution 0,24 M d'ammoniaque lentement et régulièrement. On suit le pH de la solution jusqu'à obtention d'un rapport OH/Al égal à 1,2. On arrête alors l'ajout d'ammoniaque. On chauffe la solution à 50° C, tout en agitant, pendant environ 1 heure (solution c).

Etape de pontage

On ajoute 1,4 litre de la solution c à 0,9 litre de solution b. On agite pendant 1 heure à température ambiante. On obtient la solution d.

Exemple 19 A : KMO 15 A et KMO 19 SA

La solution d obtenue précédemment est dialysée avec une membrane à dialyse en acétate de cellulose, pendant 4 jours contre 10 litres d'eau permutée changée chaque jour. L'argile pontée est alors séparée de la solution par centrifugation.

Elle est séchée à 100° C pendant 24 heures, puis calcinée à 350° C pendant 2 heures.

Distance interlamellaire :
KMO 15 A = 17,6 Angstroms (1,76 nm)
KMO 19 SA = 18,0 Angstroms (1,80 nm)
Surface spécifique BET :
KMO 15 A = 240 $m^2/g$
KMO 19 SA = 275 $m^2/g$
Volume poreux :

KMO 15 A = 0,20 $cm^3/g$
KMO 19 SA = 0,20 $cm^3/g$

Exemple 19 B : KMO 16 SA

L'argile pontée est séparée de la solution d par centrifugation. Le centrifugeat est introduit dans un ballon de 2 litres. On ajoute le volume d'eau permutée nécessaire pour obtenir une solution à 15 g/litre d'argile. On chauffe la solution ainsi obtenue à 50°C pendant 45 minutes, sous agitation à 200 tours/minute. Puis on laisse refroidir. L'argile pontée est alors séparée de la solution par centrifugation.

Elle est séchée à 100°C pendant 24 heures, puis calcinée à 350°C pendant 2 heures.
Distances interlamellaire : KMO 16 SA = 18,2 Angstroms (1,82 nm).
Surface spécifique BET : KMO 16 SA = 130 $m^2/g$
Volume poreux : KMO 16 SA = 0,12 $cm^3/g$

Exemple 19 C : KMO 20 SA

L'argile pontée est séparée de la solution d par centrifugation. On procède alors à 2 lavages successifs de l'argile, à température ambiante, de la manière suivante : le centrifuigeat est mis en suspension dans un volume d'eau permutée, de manière à avoir une solution à 15 g/litre d'argile. On agite pendant 1 heure. On sépare l'argile par centrifugation et on recommence l'opération.

L'argile pontée est alors centrifugée.

Elle est séchée à 100°C pendant 24 heures, puis calcinée à 350°C pendant 2 heures.
Distance interlamellaire : KMO 20 SA = 18,4 Angstroms (1,84 nm).
Surface spécifique BET : KMO 20 SA = 220 $m^2/g$
Volume poreux : KMO 20 SA = 0,17 $cm^3/g$

Les différents lots d'argile pontée ainsi préparés se présentent sous forme de poudre.

EXEMPLE 20 : Exemple de pontage d'une montmorillonite.

On utilise pour cet essai de pontage une montmorillonite du Wyoming, aussi appelée bentonite Volclay.

Préparation de la fraction à 2 µm de l'argile

On prépare une suspension aqueuse à 20 g/litre de cette argile, qu'on agite violemment et qu'on laisse décanter pendant 24 heures.

On fait ensemble un prélèvement de la fraction à 2 µm de l'argile (solution a).

Préparation de l'argile sous forme sodique

On ajoute à la solution a du chlorure de sodium en excès, par rapport à la capacité d'échange de l'argile, soit environ 1000 milliéquivalents de sodium pour 100 g d'argile. La solution est agitée pendant 1 heure à température ambiante. L'argile est alors filtrée de la solution. On recommence l'opération une deuxième fois. L'argile est ensuite lavée à l'eau distillée jusqu'à disparition des ions chlorures dans les eaux de lavage. L'argile est alors remise en suspension dans l'eau à 20 g/litre (solution b).

Préparation de la solution d'argile et de sel d'aluminium

On prépare une solution 0,4 M de nitrate d'aluminium. On ajoute, sous agitation et à température ambiante, 0,5 litre de cette solution de nitrate d'aluminium à 1 litre de la solution b. On agite pendant quelques minutes à température ambiante. On obtient la solution c.

Neutralisation de la solution c

A la solution c, on ajoute sous agitation et à la température ambiante une solution 0,5 M d'ammoniaque lentement et régulièrement. On suit le pH de la solution jusqu'à obtention d'un rapport OH/Al égal à 5. On arrête alors l'ajout d'ammoniaque. On poursuit l'agitation pendant environ 1 heure en chauffant légèrement la solution.

Séparation de l'argile

La solution obtenue à l'étape précédente est dialysée avec une membrane à dialyse en acétate de cellulose, pendant 4 jours contre 10 litres d'eau permutée changée chaque jour. L'argille pontée est alors séparée de la solution par centrifugation.

Elle est séchée à 100°C pendant 24 heures.
Distance interlamellaire : KMO 25 II = 14,6 Anstroms (1,46 nm)

L'argile pontée ainsi préparée se présente sous forme de poudre.

EXEMPLES 21 A 26

Dans un réacteur en verre pyrex de 30 cm3, muni d'une agitation magnétique, d'un refrigérant relié à un gazomètre, d'un système de chauffage régulé et d'un système d'injection, on charge après avoir préalablement purgé l'appareil à l'aide d'azote :
- 4,7 g de phénol (0,05 mol)
- 0,25 g d'argile pontée préparée dans l'une des exemples 1, 19 ou 20.
- 4,7 g d'un solvent (voir tableau ci-après).
- éventuellement 0,1 mmol de diphosphono-1,1 éthanol (DPE) (4 % en moles par rapport à $H_2O_2$).

On chauffe à 80° C ou 100° C sous agitation, puis on injecte en 0,1 min 0,125 g d'une solution de $H_2O_2$ à 70 % en poids par volume (2,5 mmol).

On laisse ensuite réagir pendant 2 h 30 min ou 7 heures supplémentaires.

Après filtration du catalyseur, on dose $H_2O_2$ non transformée par iodométrie et les diphénols par chromatographie liquide haute performance (CLHP).

Le tableau III ci-après rassemble les résultats obtenus (pyrocatéchine = PC ; hydroquinone = HQ).

TABLEAU III

| Ex | Argile pontée | Solvant | Durée | DPE | T°C | TT % H2O2 | RT % HQ | RT % PC | Somme RT % des diphénols |
|---|---|---|---|---|---|---|---|---|---|
| 21 | TRP-1 | MIBC * | 7 h | oui | 80 | 91,5 | 20,0 | 25,5 | 45,5 |
| 22 | KMO 20SA | eau | 2h 30 | non | 80 | 94,0 | 13,0 | 14,5 | 27,5 |
| 23 | KMO 19SA | CH3CN | 2h 30 | non | 80 | 43,5 | 8,0 | 20,5 | 28,5 |
| 24 | KMO 19SA | sulfolane | 2h 30 | non | 80 | 89,0 | 9,0 | 15,0 | 24,0 |
| 25 | KMO 25 II | eau | 2h 30 | non | 100 | 95,0 | 19,5 | 23,5 | 43,0 |
| 26 | KMO 25 II | eau | 2h 30 | oui | 100 | 97,5 | 21,0 | 25,5 | 46,5 |

* MIBC = méthylisobutylcétone.

EXEMPLES 27 à 32

Dans un réacteur en verre pyrex de 30 cm3, muni d'une agitation magnétique, d'un réfrigérant relié à un gazomètre, d'un système de chauffage régulé et d'un système d'injection, on charge après avoir préalablement purgé l'appareil à l'aide d'azote :
- 9,4 g de phénol (0,1 mole)
- 0,25 g d'argile pontée préparée dans l'un des exemples 19 ou 20.
- éventuellement 0,1 mmol de diphosphono-1,1 éthanol (DPE) (4 % en moles par rapport à $H_2O_2$).

On chauffe à 80° C sous agitation, puis on injecte en 0,1 min 0,25 g d'une solution de $H_2O_2$ à 70 % en poids par volume (5 mmol).

On laisse ensuite réagir pendant 2 h 30 min supplémentaires.

Après filtration du catalyseur, on dose $H_2O_2$ non transformée par iodométrie et les diphénols par chromatographie liquide haute performance (CLHP).

Le tableau IV ci-après rassemble les résultats obtenus (pyrocatéchine = PC ; hydroquinone = HQ).

TABLEAU IV

| Exemples | Argile Pontée | DPE | TT % H2O2 | Rt % HQ | Rt % PC | Somme RT % des diphénols |
|---|---|---|---|---|---|---|
| Ex. 27 | KMO 15 A | non | 80,0 | 10,0 | 26,0 | 36,0 |
| Ex. 28 | KMO 16 SA | non | 74,0 | 10,5 | 30,0 | 40,5 |
| Ex. 29 | KMO 19 SA | non | 89,5 | 13,0 | 25,5 | 38,5 |
| Ex. 30 | KMO 20 SA | non | 86,5 | 13,5 | 28,0 | 41,5 |
| Ex. 31 | KMO 25 II | non | 88,0 | 12,0 | 28,5 | 40,5 |
| Ex. 32 | KMO 25 II | oui | 48,5 | 21,5 | 39,0 | 60,5 |

EXEMPLE 33

Dans un réacteur en verre pyrex de 100 cm3, muni d'une agitation centrale, d'un réfrigérant relié à un gazomètre, d'un système de chauffage régulé et d'un système d'injection, on charge après avoir préalablement purgé l'appareil à l'aide d'azote :

- 36,0 g de phénol
- 0,95 g d'argile pontée préparée dans l'exemple 20 et calcinée pendant 5 heures à 550° C : KMO 25 IIA ayant les caractéristiques suivantes :
  . distance interlamellaire = 14,3 angstroms (1,43 nm)
  . surface spécifique BET = 116 m2/g
  . volume poreux = 0,14 cm3/g
- 0,0840 g de $H_3PO_4$ à 90 %.

On chauffe à 80° C sous agitation, puis on injecte en 3 minutes 0,9155 g d'une solution de $H_2O_2$ à 70 % en poids par volume.

On laisse ensuite réagit pendant 5 heures 30 supplémentaires.

Après filtration du catalyseur, on dose $H_2O_2$ non transformée par iodométrie et les diphénols par chromatographie liquide haute performance (CLHP).

On obtient les résultats suivants :

- taux de transformation (TT)de $H_2O_2$ : 91,5 %
- rendement en pyrocatéchine par rapport à $H_2O_2$ transformée (RT) : 31,0 %
- rendement en hydroquinone par rapport à $H_2O_2$ transformée (RT) : 22,5 %
- rendement total en diphénols : 53,5 %

EXEMPLE 34

On répète l'exemple 19A de préparation des argiles pontées KMO 15A et KMO 19SA, mais en n'utilisant pas uniquement la fraction à 2 µm de l'argile (solution a) mais toute la suspension.

Les caractéristiques de l'argile pontée AAY-2 ainsi préparée sont les suivantes :

Distance interlammellaire : 17,6 angstroms (1,76 nm)
Surface spécifique BET : 220 $m^2/g$
Volume poreux : 0,2 $cm^3/g$.

EXEMPLE 35

Dans un réacteur en verre pyrex de 100 cm3, muni d'une agitation centrale, d'un réfrigérant relié à un gazomètre, d'un système de chauffage régulé et d'un système d'injection, on charge après avoir préalablement purgé l'appareil à l'aide d'azote :

- 36,0 g de phénol
- 0,95 g d'argile pontée préparée dans l'exemple 34 : AAY-2.

On chauffe à 80°C sous agitation, puis on injecte en 3 minutes 0,9155 g d'une solution de $H_2O_2$ à 70 % en poids par volume.

On laisse ensuite réagir pendant 5 heures 30 supplémentaires.

Après filtration du catalyseur, on dose $H_2O_2$ non transformée par iodométrie et les diphénols par chromatographie liquide haute performance (CLHP).

On obtient les résultats suivants :

- taux de transformation (TT)de $H_2O_2$ : 94,0 %
- rendement en pyrocatéchine par rapport à $H_2O_2$ transformée (RT) : 18,0%
- rendement en hydroquinone par rapport à $H_2O_2$ transformée (RT) : 10,5 %
- rendement total en diphénols : 28,5 %

**Revendications**

1. Procédé d'hydroxylation de phénols ou éthers de phénols de formule générale (I) :

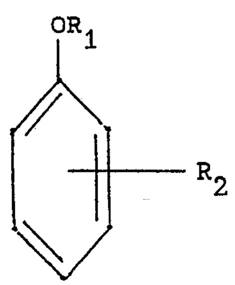

(I)

dans laquelle :
- $R_1$ représente un atome d'hydrogène, un groupement méthyle, un groupement éthyle ou un groupement phényle,
- $R_2$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace d'au moins une argile pontée.

2. Procédé selon la revendication 1, caractérisé en ce que l'argile pontée est une zéolithe à structure bidimensionnelle obtenue par un procédé consistant à opérer selon les étapes suivantes :

a) traitement d'une argile naturelle ou synthétique, comportant des substitutions en couche tétraédique, sous forme d'une suspension aqueuse, par une solution aqueuse contenant au moins un hydroxyde d'au moins un métal ;

b) l'élimination de l'excès de l'hydroxyde de métal n'ayant pas réagi sur l'argile, ladite élimination conduisant, après séchage de l'argile traitée, à l'obtention d'un précurseur de la zéolithe ;

c) le traitement thermique du précurseur de la zéolithe, ledit traitement conduisant à l'obtention de la zéolithe à structure bidimensionnelle.

3. Procédé selon la revendication 1, caractérisé en ce que l'argile pontée est préparée en mettant en oeuvre les étapes a) et b) de la revendication 2.

4. Procédé selon l'une des revendications 1 à 3 caractérisé en ce que l'argile pontée est obtenue par pontage d'une smectite.

5. Procédé selon l'une des revendications 1 à 4, caractérisé en ce que l'argile pontée est préparée à partie d'une beidellite, en particulier d'une beidellite synthétique, ou d'une montmorillonite.

6. Procédé selon l'une des revendications 1 à 5, caractérisé en ce que le pontage de l'argile est effectué à l'aide de l'une des hydroxydes des métaux suivants : l'aluminium, le nickel, le cobalt, le vanadium, le molybdène, le rhénium, le fer, le cuivre, le ruthénium, le chrome, le lanthane, le cérium, le titane, le bore, le gallium, le zirconium, le nobium, le tantale, le silicium, ou avec l'un des hydroxydes mixtes de ces métaux.

7. Procédé selon l'une des revendications 1 à 5, caractérisé en ce que le pontage de l'argile est effectué par l'hydroxyde d'aluminium.

8. Procédé selon l'une des revendications 1 à 7, caractérisé en ce qu'il est appliqué à des composés de formule (I) dans laquelle $R_1$ représente un atome d'hydrogène, un groupement méthyle ou un groupement éthyle, et $R_2$ représente un atome d'hydrogène, un groupement méthyle, éthyle ou tertiobutyle, un groupement méthoxy ou éthoxy.

9. Procédé selon l'une des revendications 1 à 8, caractérisé en ce qu'il est appliqué aux phénols et éthers de phénols choisis parmi le phénol, l'anisole, l'orthocrésol, le métacrésol, le paracrésol, le

tertiobutyl-4 phénol, le méthoxy-2 phénol, le méthoxy-4 phénol.

10. Procédé selon l'une des revendications 1 à 9, caractérisé en ce que le rapport molaire composé de formule I/peroxyde d'hydrogène est de 25/1 a 3/1 et de préférence de 20/1 à 4/1.

11. Procédé selon l'une des revendications 1 à 10 réalisé en discontinu, caractérisé en ce que le catalyseur représente en poids par rapport au poids du composé de formule I engagé de 0,1 % à 20 % et de préférence de 0,5 % à 10 %.

12. Procédé selon l'une des revendications 1 à 10, caractérisé en ce que le procédé est réalisé en continu sur un lit fixe de catalyseur.

13. Procédé selon l'une des revendications 1 à 12, caractérisé en ce que le peroxyde d'hydrogène est mis en oeuvre sous la forme d'une solution aqueuse.

14. Procédé selon l'une des revendications 1 à 12, caractérisé en ce que le peroxyde d'hydrogène est mis en oeuvre sous la forme d'une solution organique.

15. Procédé selon l'une des revendications 1 à 14, caractérisé en ce que la réaction d'hydroxylation est effectuée dans un solvant du composé de formule I, de préférence miscible ou partiellement miscible à l'eau, tel que l'eau, un alcool, une cétone, un nitrile, un acide carboxylique, un ester, un éther ou un solvant aprotique polaire.

16. Procédé selon l'une des revendications 1 à 14, caractérisé en ce que l'on opère en présence de 0 à 10 % en moles d'un acide phosphorique, d'un acide phosphonique ou de l'un de leurs dérivés, par rapport au peroxyde d'hydrogène.

17. Procédé selon l'une des revendications 1 à 16, caractérisé en ce que la réaction d'hydroxylation est conduite à une température comprise entre 45° C et 160° C.